# EUROPEAN PATENT APPLICATION

(11) **EP 3 995 829 A1**
(43) Date of publication of application: **11.05.2022**
(21) Application number: 20206478.8
(22) Date of filing: 09.11.2020
(51) Int. Cl.: G01N 33/569

(54) **LGALS3BP AS A DIAGNOSTIC, PROGNOSTIC, AND THERAPEUTIC BIOMARKER IN COVID-19 AND OTHER CYTOKINE STORM CONDITIONS**

(71) Applicant: MediaPharma S.r.l., 66100 Chieti (IT)
(72) Inventor: GENTILE, Roberta, 67100 L'Aquila (IT); IACOBELLI, Valentina, 00159 Roma (IT); IACOBELLI, Stefano, 00198 Roma (IT); SAUNIERE, Jean Frédéric Michel Leo, 83110 Sanary (FR); NATALI, Pier Giorgio, 00141 Rome (IT); PIANTELLI, Mauro, 00167 Rome (IT)
(74) Representative: Heubeck, Christian

(57) **Abstract**

The present invention relates generally to the identification of patients with COVID-19 disease and other cytokine storm diseases as to whether they will progress to advanced stage of the disease. More particularly, the invention relates to the use of Gal-3BP in biological fluids and tissues/cells, preferably saliva as a biomarker for early identification of COVID-19 patients who progress to Acute Respiratory Distress Syndrome (ARDS) requiring mechanical ventilation.

## Description

### FIELD OF THE INVENTION

The present invention relates generally to the identification of patients with COVID-19 disease and other cytokine storm diseases as to whether they will progress to advanced stage of the disease. More particularly, the invention relates to the use of Gal-3BP in biological fluids and tissues/cells, preferably saliva as a biomarker for early identification of COVID-19 patients who progress to Acute Respiratory Distress Syndrome (ARDS) requiring mechanical ventilation.

### BACKGROUND OF THE INVENTION

"A biomarker is a biological characteristic, which can be molecular, anatomic, physiologic, or biochemical. These characteristics can be measured and evaluated objectively. They act as indicators of a normal or a pathogenic biological process. They allow assessing the pharmacological response to a therapeutic intervention. A biomarker shows a specific physical trait or a measurable biologically produced change in the body that is linked to a disease or a particular health condition." (European Commission, DG Research - Brussels, 2010).

Biomarkers may be found, for example, in blood or other body fluids as well as tissues. Biomarkers can be differentiated into predictive and prognostic biomarkers. Predictive biomarkers are used to assess the probability that a patient will respond to or benefit from a particular treatment. Prognostic biomarkers allow for the classification of pathological condition or disease with regard to their progression and to guide a decision of whom to treat or how to treat.

Severe acute respiratory syndrome coronavirus 2 (SARS-CoV-2) is the strain of coronavirus that causes the novel coronavirus disease 2019 (COVID-19), which is the respiratory illness responsible for the global COVID-19 pandemic. Nearly 50 million verified infections and over 1 million deaths have been reported globally so far. These numbers are very likely to increase markedly during the near future, challenging the capacity of healthy systems worldwide. The spectrum of COVID-19 disease can range from mild asymptomatic infection to severe pneumonia with acute respiratory distress syndrome (ARDS) and death.

Taken this variability of COVID-19 disease and the limitation of health care resources in heavily affected regions, it is of crucial importance to classify patients in need of Intensive Care Units (ICU). Such classification would allow efficient allocation of resources towards patients at high risk for deterioration. In this context, the use of biomarkers could identify early stages of progression toward ARDS and/or death that is fundamental for the practical management of COVID-19 patients.

The main cause of death in COVID-19 patients is linked to the onset of bilateral interstitial pneumonia, of varying severity, which may require access to ICU and mechanical ventilation. Patients with pneumonia often develop ARDS. ARDS often requires treatment in the form of mechanical ventilation. Among hospitalized COVID-19 patients, approximately 33% develop ARDS, 26% require transfer to an ICU and 16% receive some form of mechanical ventilation (1).

For patients infected with SARS-CoV-2, it has been described that severe ARDS and clinical outcome are related to the characteristics of the immune response (2). Interleukin (IL)-6 and other components of the inflammatory cascade contribute to host defense against infections. However, exaggerated synthesis of IL-6 can lead to an acute severe systemic inflammatory response known as 'cytokine storm' which is associated with serious adverse outcomes in patients infected with SARS-CoV-2 pneumonia (3). Several reports have documented that IL-6 serum levels are a good prognosticator for progression to ARDS and/or mortality (4,5).

In view of the above, it is an object of the present invention to provide a method for predicting whether a COVID-19 patient will progress to ARDS requiring mechanical ventilation. It would be particularly advantageous to have a method that is simple to carry out and is based on the measurement of a marker in a biological fluid.

### DESCRIPTION OF THE INVENTION

According to the present invention, it was found that level and or concentration of Gal-3BP may provide information to assess whether a patient with COVID-19 or other cytokine storm diseases is expected to have a severe course of the disease.

Thus, the present invention provides a method for predicting whether a patient with COVID-19 disease and other cytokine storm diseases will progress to an advanced disease stage by determining a biomarker present in biological fluids.

The human Galectin-3-binding protein (Gal-3BP, Uniprot ID - Q08380), also known as 90K, Mac-2 BP or LGALS3BP is a heavily glycosylated, large oligomeric, human protein consisting of about 90 kDa subunits. The protein was originally isolated from the conditioned medium of human breast cancer cells (8,9). It is expressed on several cell types, mostly in cavity lining respiratory and gastrointestinal epithelial cells (10).

Functionally, Gal-3BP has been shown to mediate adhesive processes, including homotypic cell adhesion and cell to extra-cellular matrix (ECM) adhesion (11,12). Recently, the protein has been shown to stimulate tumor angiogenesis through a pathway independent of that used by Vascular Endothelial Growth Factor (VEGF) (13).

Moreover, several research groups have documented that Gal-3BP induces IL-6 expression and secretion in multiple different cell types (14-17) The induction requires carbohydrate-mediated interaction between Gal-3BP and Galectin-3 at the cell surface and involves the MAPK/ERK pathway (14,17).

Gal-3BP is found in the serum and other body fluids, including milk, saliva, tears, semen of apparently healthy individuals (18-20). However, Gal-3BP serum concentrations and expression levels in tissues are significantly elevated in several neoplastic pathologies and predict poor clinical outcome, for instance in breast cancer (21, 22) and lung cancer (23).

Additionally, supranormal Gal-3BP levels have been documented in patients with viral infection. First indications of the role of Gal-3BP in viral infections were obtained when a group of scientists observed patients with HIV-associated Kaposi's sarcoma to have unusually high Gal-3BP serum concentrations (24, 25). Additionally, a negative correlation between Gal-3BP concentration and CD4+ cell count in HIV-infected patients, but a positive correlation between Gal-3BP and HIV p24 antigen concentrations was observed (24, 25). A subsequent retrospective study with HIV-infected patients showed that increases in Gal-3BP concentration preceded the decrease in CD4+ cells, suggesting a prognostic value for Gal-3BP concentration in HIV infection towards AIDS (26). Indeed, high Gal-3BP concentrations in HIV-infected individuals were associated with faster progression towards AIDS in follow-up studies (27-29). Overall, the above data indicate that the levels of Gal-3BP assayed in different categories of asymptomatic HIV-infected patients are an independent, prognostic factor of decrease of CD4+ cell counts and faster development of AIDS.

Thus, according to a first aspect, the present invention relates to an *in vitro* method for predicting or monitoring whether a patient suffering from COVID-19 or other cytokine storm diseases will deteriorate and progress to an advanced stage of the disease, in particular Acute Respiratory Distress Syndrome (ARDS) requiring mechanical ventilation, by evaluating a biomarker in a sample from the patient, wherein the biomarker is Gal-3BP.

According to a preferred embodiment, the present invention is directed to an *in vitro* method for determining the prognosis of a COVID-19 disease or other cytokine storm disease in a SARS-CoV-2 infected patient comprising the following steps:
(i) assessing the level of expression and/or concentration of Gal-3BP in a sample obtained from said patient, preferably COVID-19 disease patient;
(ii) classifying the patient, preferably COVID-19 disease patient, as having a risk of progressing to ARDS requiring mechanical ventilation if the level of Gal-3BP is > 300 ng/ml bodyfluid, preferably > 500 ng/ml body fluid, and optionally
(iii) initiating prevention measures and/or a treatment of pathological effects of the SARS-CoV-2 infection, such as administering at least one drug for the prevention and or treatment of cytokine storm diseases, in particular ARDS, if the patient has a risk of progressing to ARDS as determined in steps (i) and (ii).

According to this invention, "a patient" may be every person which is SARS-CoV-2 infected, i.e. any person that has been positively tested for a SARS-CoV-2 infection, regardless if the person shows any typical infection symptoms, or a person which has not been positively tested for SARS-CoV-2 infection, but shows typical infection symptoms like fever, cough, sore throat, headaches, pneumonia, loss of taste or smell etc.

Within the meaning of the present invention *"SARS-CoV-2"* designates the new Coronavirus causative of the *"COVID-19 disease"* as well as any mutations thereof.

According to a preferred embodiment of the invention, the inventive method of determining the prognosis of a COVID-19 disease comprises the initial step of testing a person for SARS-CoV-2 infection. Suitable methods are known to the person skilled in the art and comprise PCR based methods as well as immunological methods, in particular methods detecting SARS-CoV-2 antigen. If the tested person shows a SARS-CoV-2 infection, the steps of the inventive method are to follow.

Thus, according to a further embodiment, the present invention relates to an *in vitro* method for determining the prognosis of a COVID-19 disease or other cytokine storm disease in a SARS-CoV-2 infected patient comprising the following steps:
(i) testing a person for SARS-CoV-2 infection, for example by PCR and/or immunological based methods, and if the person shows a positive test result for SARS-CoV-2 infection,
(ii) assessing the level of expression and/or concentration of Gal-3BP in a sample obtained from said patient, i.e. person showing a positive test result for SARS-CoV-2 infection;
(iii) classifying the patient as having a risk of progressing to ARDS requiring mechanical ventilation if the level of Gal-3BP is > 300 ng/ml body fluid, preferably > 500 ng/ml body fluid, and optionally
(iv) initiating prevention measures and/or a treatment of pathological effects of the SARS-CoV-2 infection, such as administering at least one drug for the prevention and or treatment of cytokine storm diseases, in particular ARDS, if the patient has a risk of progressing to ARDS as determined in steps (ii) and (iii).

The sample to be tested for its Gal-3BP level and/or concentration may be taken from any body fluid (such as blood, milk, saliva, tears, semen) or tissues, (such as the mucosa of oral cavity, nasal cavities, pharynx, larynx) or any kind of cells, (such as epithelial cells lining the oral cavity, the pharynx and larynx).

In COVID-19 patients, the levels of Gal-3BP in saliva may give information on the risk whether they will progress to ARDS requiring mechanical ventilation. Thus, according an especially preferred embodiment of the invention the sample to be tested is saliva, i.e. COVID-19 patients have a risk of progressing to ARDS, in particular ARDS requiring mechanical ventilation if the their level of Gal-3BP is > 300 ng/ml saliva, preferably > 500 ng/ml saliva.

*"Cytokine storm",* also called hypercytokinemia within the meaning of the present disclosure shall be understood as a physiological reaction in humans and other animals in which the innate immune system causes an uncontrolled and excessive release of pro-inflammatory signaling molecules called cytokines. Normally, cytokines are part of the body's immune response to infection, but their sudden release in large quantities can cause multisystem organ failure and death (30). Other than by SARS-CoV-2, cytokine storms can be caused by a number of infectious and non-infectious etiologies, especially viral respiratory infections such as H5N1 influenza, SARS-CoV-1 (31, 32). Other causative agents include the Epstein-Barr virus, cytomegalovirus, and group A streptococcus, and non-infectious conditions such as graft-versus-host disease (33).

*"Cytokine storm diseases"* within the meaning of the present disclosure shall be understood as a diverse set of pathological conditions, such as familiar hemophagocytic lymphohistiocytosis, Epstein-Barr virus-associated hemophagocytic lymphohistiocytosis, systemic or non-systemic juvenile idiopathic arthritis-associated macrophage activation syndrome, NLRC4 macrophage activation syndrome, cytokine release syndrome and sepsis (34), which are consequences of a cytokine storm.

*"ARDS requiring treatment"* within the meaning of the present disclosure shall be understood as a respiratory failure condition that involves mechanical ventilation together with treatments directed at the underlying cause (35). Ventilation strategies include using low volumes and low pressures (35).

Drugs to be administered for the prevention and or treatment of ARDS and/or cytokine storm diseases may be, for example, corticosteroids, heparin, anti-coagulant and antiplatelet therapies, statins, thiol-based drugs (N-acetylcysteine, NAC), tocilizumab (anti-IL-6R antagonist) (6), Neurokinin-1 (NK-1) receptor antagonists, antiviral agents, such as Molnupiravir, Colony-stimulator factors, Mesenchymal stem cells, Phosphodiesterase inhibitors (7).

A further object of the present disclosure is the use of Gal-3BP as a biomarker for selecting those COVID-19 patients at risk of ARDS likely to benefit from therapies aiming to prevent or to counteract the pathological effects of the infection.

Preferably, the in vitro assay of the present invention of the Gal-3BP is performed to foresee the evolution of the SARS-CoV-2 infected patients to ARDS requiring mechanical ventilation, to monitor the progress of SARS-CoV-2 infection and to evaluate the efficacy of therapies aiming at contrast the pathological effects of the SARS-CoV-2 infection or to alleviate the accompanying symptoms.

In another embodiment, the present invention discloses that targeting Gal-3BP, which is known to induce IL-6 may inhibit the initiation/reduce the intensity of the cytokine storm provoked by SARS-CoV-2, therefore avoiding progression to ARDS and improving outcomes in COVID-19 patients. Gal-3BP may be targeted by specific antibodies or by products/agents, such peptides, glyco-peptides or glyco-mimetics, etc. able to interfere with Gal-3BP-induced IL-6 production. Antibody-drug conjugates capable of interfering with Gal-3BP are described in WO 2019/197651. Thus, according to this this invention, Gal-3BP targeting drugs, such as Gal-3BP antagonists, are preferred drugs to prevent or treat a cytokine storm and/or ARDS.

The method to assay Gal-3BP may comprise any immunological procedures known to the person skilled in the art, such as ELISA, EIA, LFA, IRMA, or LC/MS procedures, or western blotting procedures. Especially preferred is Lateral Flow Assay (LFA). LFAs, also called lateral flow immunochromatographic assays, are based on simple devices intended to detect the presence of a target substance in a liquid sample without the need for specialized and costly equipment. These tests are widely used in medical diagnostics for home testing, point of care testing, or laboratory use. For instance, the home pregnancy test is a lateral flow test that detects a certain hormone

It is intended that the immunological procedures intended to be used herein may comprise an antibody against Gal-3BP, i.e. an anti-Gal-3BP antibody. In general, any antibody or antibody fragment binding Gal-3BP can be used.

The term *"antibody"* particularly refers to molecules comprising at least one immunoglobulin heavy chain and at least one immunoglobulin light chain. Each heavy and light chain may comprise a variable and a constant domain. The antigen binding site may be formed from the variable domains of a heavy chain and a light chain. A variable region (also referred to as variable domain) comprises complementarity determining regions (CDRs), e.g. a CDR1, a CDR2 and a CDR3 region and framework regions (FRs) flanking the CDRs.

The present invention encompasses both full length immunoglobulin and functional immunoglobulin fragments like Fab, Fab', F(ab')2 fragments, Fv fragments, diabodies, single-chain antibody molecules and single-domain antibodies. An *"antibody fragment"* according to the invention presents essentially the same epitope binding site as the corresponding antibody. *"Diabodies"* are antibody fragments with two antigen-binding sites that may be bivalent or bispecific. *"Single-chain antibodies"* are antibody fragments comprising all or a portion of the heavy chain variable domain, or all or a portion of the light chain variable domain of an antibody. Antibody fragments can be made by various techniques, including but not limited to proteolytic digestion of an intact antibody as well as production by recombinant hosts (e.g., *E-coli* or phage).

Methods for producing antibodies of the invention are known to the person skilled in the art.

According to the invention, antibodies may be used with or without modification, and may be labelled, either covalently or non-covalently, with, for example, a reporter group or an effector group. The antibodies used in the immunological procedures used herein are preferably labelled, for example, by biotin-labels, gold-labels, radioactive-labels, enzyme labels, fluorescent labels etc. and/or mixtures thereof. The use of biotin and/or gold labelled antibodies is in particular preferred.

An especially preferred antibody is the monoclonal antibody SP-2 or any Gal-3BP binding fragment thereof. SP-2 was purified from the correspondent hybridoma cell line, deposited at DSM DEUTSCHE SAMMLUNG VON MIKROORGANISMEN UND ZELLKULTUREN GmbH under the Budapest Treaty under the accession number DSM ACC2116, Mascheroder Weg 1 B D-3300 Braunschweig, Germany, and at C.N.C.M. the Collection Nationale de Cultures de Microorganismes at the Pasteur Institute, Paris, France, under the accession number I-1083, as previously described by the present inventor, Iacobelli et al., 1986, Cancer Research, 46, 3005-3010 and Iacobelli et al., 1988, Breast Cancer Research and Treatment, 11, 19-30.

Another aspect of the present invention relates to a kit for classifying a COVID-19 patient as having a risk of progressing to ARDS requiring mechanical ventilation comprising Gal-3BP targeting antibodies and/or antibody fragments and a testing device for carrying out an LFA, such as a test strip. The antibodies of the invention may be immobilized on the test strip. The test strip may comprise an indicator dye, i.e. a dye which shows a color, staining or change of color if a certain threshold value is reached, i.e. if the Gal-3BP level concentration is above 300 ng/ml. According to a preferred embodiment, the Gal-3BP antibody is the SP-2 antibody described herein and/or a fragment thereof, preferably biotin and/or gold labelled

### BRIEF SUMMARY OF THE FIGURES

**FIG. 1****:** Box plot of Gal-3BP in saliva of COVID-19 patients. The dashed line refers to the Gal-3BP threshold of > 300ng/ml.
**FIG. 2****:** Receiver operating characteristic (ROC) curve of salivary Gal-3BP levels before mechanical ventilation, AUC=0.92.

### EXPERIMENTAL DATA

In total, 38 hospitalized patients with PCR (polymerase chain reaction) proven SARS-Cov-2 infected (COVID-19 patients) were selected for the study. Patients were analyzed for baseline clinical and laboratory findings. Decision on endotracheal intubation was made following internationally accepted recommendations (PaO₂/FiO₂ <150mmHg) (36). Patient data were anonymized for analysis and the study was approved by the local ethics committee. *"PaO₂*/*FiO₂ ratio"* (ratio of partial pressure arterial oxygen and fraction of inspired oxygen) within the meaning of the present disclosure shall be understood as the ratio of partial pressure arterial oxygen and fraction of inspired oxygen.

In total, 12/38 (31.5%) patients developed ARDS during hospitalization and required mechanical ventilation after a median time of 3.5 days. There were no differences in age, comorbidities, radiological findings, respiratory rate between patients requiring or not mechanical ventilation.

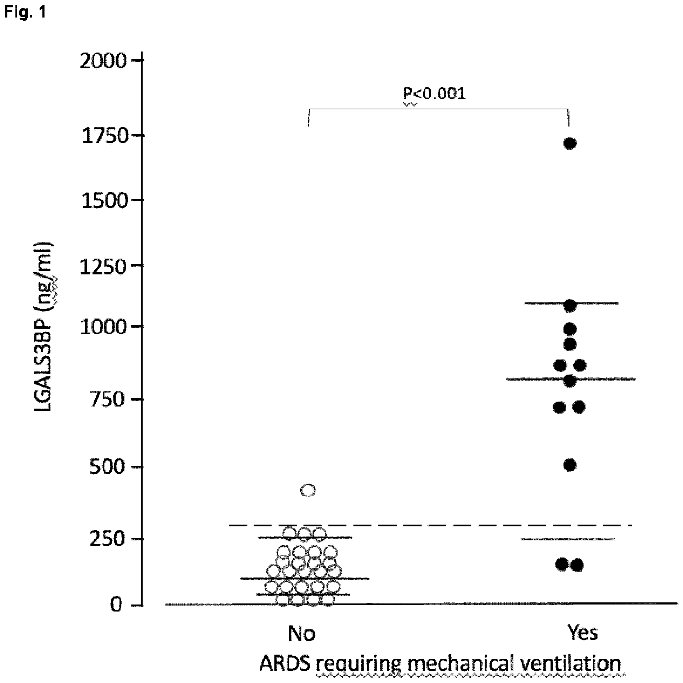

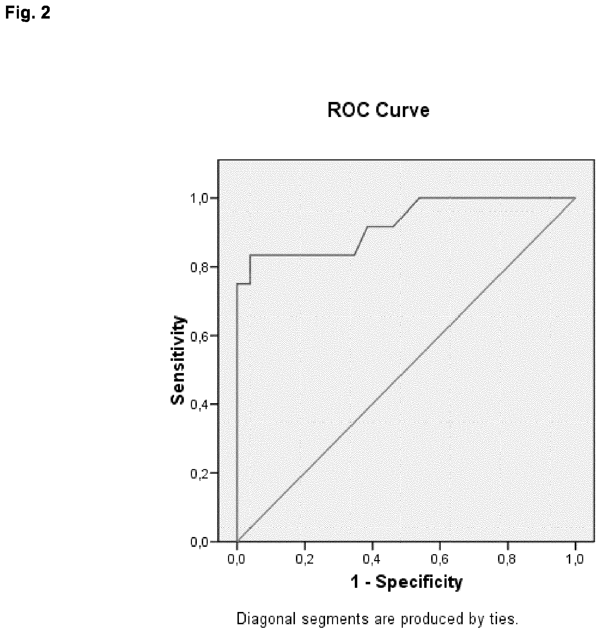

Surprisingly, levels of salivary Gal-3BP measured at the time of hospitalization were significantly associated with the need of mechanical ventilation (Fig. 1; p<0.001). Moreover, Gal-3BP proved to be an accurate classifier of COVID-19 disease (Fig. 2, p<0.001; AUC=0.92). The statistically optimal cutoff for salivary Gal-3BP was 300 ng/ml. This cutoff identified all but two patients with respiratory failure and misclassified one patient without respiratory failure.

### Materials and Methods

### Saliva Sample Collection and Handling

Patients were asked not to eat, drink, or use saliva stimulators such as chewing gum or mint for at least 1 h before sampling. Saliva was collected by the passive drool technique using a normal drinking straw about 3 cm long, directly inserted into 1.5 ml polypropylene tubes containing 0.25 ml of 0.1% sodium azide in Phosphate buffered saline, pH 7.4. during 1-2 minutes without swallowing. The saliva samples were frozen at -20°C, until analysis. All samples were centrifuged at 4500 x g for 15 min. Supernatants were drawn off and used in the assay of Gal-3BP.

### Assay of Gal-3BP in saliva

### Enzyme-Linked Immunosorbent Assay (ELISA).

The assay of Gal-3BP was performed using the 90K/Mac-2BP Monoclonal Antibody (SP-2) kit (eBioscience^{™} Catalog # BMS146), following the manufacturer's recommendations.

### Quantitative Lateral Flow Assay (LFA)

Recombinant Gal-3BP stock solutions for calibration with concentrations of 50, 100, 200, 400, 800, and 1600 ng/ml were prepared in PBS pH 7.4. Every calibration solution was measured in three repetitions. Recombinant Gal-3BP was isolated and purified as described (37).

### Conjugation of the anti-Gal-3BP antibody SP-2 with Gold.

Naked Gold Conjugation Kit (BioPorto Diagnostics A/S) is an easy-to-use kit for preparing highly reactive gold conjugates. According to manufacturer's recommendations, SP-2 (1 mg/ml) was dialysed in ultra-highly pure water overnight at 4 °C. The Naked Gold Sol is negatively charged and nanogold particles remain in solution because they repel each other. For optimal conjugation, the coating pH was maintained slightly above the isoelectric point of the antibody. Therefore, varying amounts of buffers A, B, C and D (with pH-values between 5.4 and 10.1) contained in the kit were mixed with 0.5 ml of colloidal gold and 30 mg/ml of SP-2 antibody at room temperature for 30 minutes. To test the effectiveness of the conjugation reaction, a salt test was performed, with the use of 10 ml of 1mol/L NaCl solution mixed in a 1:1 ratio with 10 µl of coated gold sol at different pH(s). Sols with incomplete coating felt out of solution (turn black), while completely coated sols remained stable (red).

### Antibody SP-2 Biotinylation

SP-2 (1mg/ml) was dialysed in PBS buffer, pH 7.4 overnight at 4 °C. Then, SP-2 was incubated with NHS-PEG4-Biotin kit (Thermo Fischer Scientific) for 30 minutes at room temperature. The desalting column provided in the kit was centrifugated at 1500 x g for 1 minute to remove the storage solution and then washed three times with 300 ml of PBS (1500 x g for 1 min). Finally, it was applied to the NHS-PEG-Biotin/Antibody column and centrifuged at 1500 x g for 2 minutes. The collected conjugate was stored at 4°C.

### Execution of the assay

The gRAD (generic Rapid Assay Device, BioPorto Diagnostics) is an optimized generic LFA strip with two printed lines where the migration occurs by capillary force: a test line composed of biotin-binding protein that binds the "sandwich" complex formed by biotinylated SP-2/Gal-3BP/gold-labeled SP-2 and a control line that captures the unreacted gold-labeled SP-2.

For the test, 5 µl biotinylated of SP-2 (0.2 mg/ml), 5 µl of SP-2 Gold Antibody and 100 µl of Gal-3BP calibration solution or salivary sample in PBS were mixed in a broad-bottom tube. After 5 minutes of incubation at room temperature, the end of the strips were placed in the above mix. After 10 minutes, the mix diffused by capillarity along the strips, coloring the control Line and the test Line, if the sample is positive.

There are several options to measure the intensity of the test line to determine the quantity of analyte (Gal-3BP) in the sample (38). Handheld diagnostic devices known as lateral flow readers are used by several companies to provide a fully quantitative assay result. By utilizing unique wavelengths of light for illumination in conjunction with either complementary metal oxide semiconductor (CMOS) or charge coupled device (CCD) detection technology, a signal rich image can be produced of the actual test lines. Using image processing algorithms specifically designed for a particular test type and medium, line intensities can then be correlated with analyte concentrations. One such handheld lateral flow device platform is made by Detekt Biomedical L.L.C. (39). Mobile phones have demonstrated to have a strong potential for the quantification in lateral flow assays. The method described below to quantify Gal-3BP in LFA uses a smart phone 5S smartphone readout that was originally applied for the monitoring of digoxigenin (40).

The test-strips were then placed on a flat surface and allowed to dry for 10 min. Images of lateral flow strips were taken with an iPhone 5S and analyzed by the R-package GNSplex, which also includes a Shiny app for quantitative computation of Gal-3BP concentrations for potential use as a point-of-care (POC)-device at home.

Such salivary LFA tests can be applied readily in a variety of settings and for specific measurement purposes, providing researchers and clinicians with opportunities to assess biomarkers in real time with lower transportation, collection, and analysis costs, faster turnaround time, and minimal training requirements. Specially, these tests can be carried out in non-laboratory environments.

In conclusion, our invention shows that Gal-3BP levels in saliva of SARS-CoV-2 infected patients is an effective biomarker to predict upcoming respiratory failure with high accuracy and help physicians correctly allocate patients at an early stage. a testing device for carrying out an LFA, such as a test strip.

### REFERENCES

1. Tzotzos S et al. Incidence of ARDS and outcomes in hospitalized patients with COVID-19: a global literature survey. Crit Care. 2020; 24: 516
2. Yang L. et al. COVID-19: immunopathogenesis and Immunotherapeutics. Signal Transduction and Targeted Therapy volume 5, Article number: 128 (2020)
3. Ragab D et al. The COVID-19 Cytokine Storm; What We Know So Far. Published online 2020 Jun 16. doi: 10.3389/fimmu.2020.01446
4. Grifoni E et al. Interleukin-6 as prognosticator in patients with COVID-19. J Infect. 2020, 81: 452-482.
5. Herold T et al. Level of IL-6 predicts respiratory failure in hospitalized symptomatic COVID-19 patients. Journal of Allergy and Clinical Immunology doi: 10.1016/j.jaci.2020.05.008
6. Matera MG, Pharmacological management of COVID-19 patients with ARDS (CARDS): A narrative review. Respir Med. 2020; 171: 106114.
7. https://www.medscape.com/answers/2500114-197461/what-other-drugs-are-being-investigated-to-treat-ardscytokine-release-associated-with-coronavirus-disease-2019-covid-19.
8. Iacobelli S et al. Detection of anti- gens recognized by a novel monoclonal antibody in tissue and serum from patients with breast cancer. Cancer Res. 1986;46: 3005-3010.
9. Iacobelli S et al. Purification and characterization of a 90 kDa protein released from human tumors and tumor cell lines. FEBS Lett. 1993;319: 59-65.
10. Ullrich A et al. The secreted tumor-associated antigen 90K is a potent immune stimulator. J Biol Chem,1994, 28:18401-18407
11. Inohara H, Raz A. Identification of human melanoma cellular and secreted ligands for galectin-3. Biochem Biophys Res Commun. 1994;201:1366-1375
12. Sasaki T et al. Mac-2 binding protein is a cell-adhesive protein of the extracellular matrix which self-assembles into ring-like structures and binds beta 1 integrins, collagens and fibronectin. EMBO J. 1998 Mar 16; 17(6): 1606-1613.
13. Piccolo E et al. LGALS3BP, lectin galactoside-binding soluble 3 binding protein, induces vascular endothelial growth factor in human breast cancer cells and promotes angiogenesis. J Mol Med (Berl) 2013 Jan;91(1):83-94.
14. Fukaya Y et al. Identification of galectin-3-binding protein as a factor secreted by tumor cells that stimulates interleukin-6 expression in the bone marrow stroma. J Biol Chem. 2008;283:18573-18581
15. Kalayci O et al. Role of 90K protein in asthma and TH2-type cytokine expression. Ann Allergy Asthma Immunol. 2004;93:485-492.
16. Powell TJ et al. A tumor-derived protein which provides T-cell costimulation through accessory cell activation. J Immunother Emphasis Tumor Immunol. 1995;17:209-221.
17. Silverman AM et al. A galectin-3-dependent pathway upregulates interleukin-6 in the microenvironment of human neuroblastoma. Cancer Res. 2012;72:2228-2238.
18. Iacobelli S et al. Detection of anti- gens recognized by a novel monoclonal antibody in tissue and serum from patients with breast cancer. Cancer Res. 1986;46: 3005-3010.
19. Koths K et al. Cloning and characterization of a human Mac-2-binding protein, a new member of the superfamily defined by the macrophage scavenger receptor cysteine- rich domain. J Biol Chem. 1993;268:14245-14249.
20. Fornarini B et al.Human milk 90K (Mac-2 BP): possible protective effects against acute respiratory infections. Clin Exp Immunol. 1999;115: 91-94.
21. Iacobelli S et al. Prognostic value of a novel cir- culating serum 90K antigen in breast cancer. Br J Cancer. 1994;69: 172-176.
22.40. Tinari N et al. High expression of 90K (Mac-2 BP) is associated with poor survival in node-negative breast cancer patients not receiving adjuvant systemic therapies. Int J Cancer. 2009;124:333-338.
23. Marchetti A et al. Expression of 90K (Mac-2 BP) correlates with distant metastasis and predicts survival in stage I non- small cell lung cancer patients. Cancer Res. 2002;62:2535-2539.
24. Iacobelli S et al. Lipoprotein 90K in human immunodeficiency virus-infected patients: a further serologic marker of progression. J Infect Dis. 1991;164:819.
25. Natoli C et al. Unusually high level of a tumor- associated antigen in the serum of human immunodeficiency virus- seropositive individuals. J Infect Dis. 1991;164:616-617.
26. Natoli C et al. 90K protein: a new predic- tor marker of disease progression in human immunodeficiency virus infection. J Acquir Immune Defic Syndr. 1993;6:370-375.
27. Briggs NC et al, Iacobelli S. A 90-kDa protein serum marker for the prediction of progression to AIDS in a cohort of HIV-1+ homosexual men. AIDS Res Hum Retroviruses 1993;9:811-816.
28.71. Natoli C, Ortona L, Tamburrini E, et al. Elevated serum levels of a 90,000 daltons tumor-associated antigen in cancer and in infection by human immunodeficiency virus (HIV). Anticancer Res. 1994;14:1457-1460.
29.72. Iacobelli S, Ullrich A, Tinari N, et al. The 90K tumor-associated antigen and clinical progression in human immunodeficiency virus infection. J Acquir Immune Defic Syndr Hum Retrovirol. 1995;10: 450-456.
30. Farsalinos K et al. (2020). Systematic review of the prevalence of current smoking among hospitalized COVID-19 patients in China: Could nicotine be a therapeutic option? Internal and Emergency Medicine. 15 (5): 845-852.
31. Wong J et al. (2017). Current and future developments in the treatment of virus-induced hypercytokinemia. Future Medicinal Chemistry. 9 (2): 169-178.
32. Qiang L et al. (January 2016). The cytokine storm of severe influenza and development of immunomodulatory therapy. Cellular & Molecular Immunology. 13(1): 3-10.
33. Tisoncik J et al. (2012). Into the Eye of the Cytokine Storm. Microbiology and Molecular Biology Reviews. 76 (1): 16-32
34. Behrens E et al. (2017). Review: Cytokine Storm Syndrome: Looking Toward the Precision Medicine Era. Arthritis & Rheumatology. 69 (6): 1135-1143.
35. Brodie FE et al. (2018) Acute Respiratory Distress Syndrome: Advances in Diagnosis and Treatment. JAMA. 319 (7): 698-710.
36. Bellani G, Laffey JG, Pham T, et al. Epidemiology, Patterns of Care, and Mortality for Patients With Acute Respiratory Distress Syndrome in Intensive Care Units in 50 Countries. JAMA. 2016; 315:788-800
37. Piccolo E et al. Prognostic relevance of LGALS3BP in human colorectal carcinoma. J Transl Med. 2015; 13: 248.
38. Urusov E et al: Towards Lateral Flow Quantitative Assays: Detection Approaches: Biosensors (Basel). 2019 Sep; 9(3): 89.
39. Detekt Biomedical L.L.C.- Lateral Flow Readers for Rapid Test Strip Detection and Immunoassays. idetekt.com. Retrieved 2017-07-06.
40. Ruppert C et al. A smartphone readout system for gold nanoparticle-based lateral flow assays: application to monitoring of digoxigenin. Microchimica Acta 186: 119 (2019).

## Claims

1. An *in vitro* method for predicting or monitoring whether a patient suffering from COVID-19 or other cytokine storm diseases will deteriorate and progress to an advanced stage of the disease, in particular Acute Respiratory Distress Syndrome (ARDS) requiring mechanical ventilation, by evaluating a biomarker in a sample from the patient, wherein the biomarker is Gal-3BP.

2. The *in vitro* method of claim 1, comprising the steps
(i) determining the concentration of Gal-3BP in a fluid sample obtained from the patient,
(ii) classifying the patient as having a risk of progressing to ARDS, in particular ARDS, requiring mechanical ventilation, if the level of Gal-3BP is > 300 ng/ml and optionally
(iii) administering at least one drug for the prevention and or treatment of cytokine storm diseases, in particular ARDS, and/or preparing ventilation strategies if the patient has a risk of progressing to ARDS as determined in steps (i) and (ii).

3. The *in vitro* method of claims 1 or 2, wherein the body fluid is saliva.

4. The *in vitro* method of any of claims 1 to 3, wherein the Gal-3BP concentration is measured by an immunological procedure, preferably Lateral Flow Assay (LFA).

5. The *in vitro* method of claim 4, wherein the immunological procedure is based on the use of anti-Gal-3BP antibodies.

6. The *in vitro* method of any of claims 1 to 5, wherein the anti-Gal-3BP antibody is SP-2 and/or any Gal-3BP binding fragments thereof.

7. The *in vitro* method of claim 1, wherein the sample comprises biological fluids, such as blood, tears, semen and in particular preferred saliva.

8. The *in vitro* method of claim 1, wherein the sample comprises tissues of the respiratory tract including those of oral cavity, nasal cavities, pharynx and larynx.

9. The *in vitro* method of claim 1, wherein the sample comprises cells, such as epithelial cells lining the oral cavity, the pharynx and larynx.

10. The *in vitro* method of any one of claims 7 to 9, wherein the Gal-3BP level and/or Gal-3BP concentration, preferably salivary GAL-3BP level and/or Gal-3BP concentration, is measured by immunological procedures, such as Enzyme-linked Immunosorbent Assay (ELISA), Enzyme Immmunoassay (EIA), immunoradiometric assay (IRMA), Lateral Flow Assay (LFA), or Liquid chromatography-mass spectrometry (LC-MS) procedures or Western blotting.

11. The method of any one or the preceding claims, wherein Gal-3BP levels, preferably salivary Gal-3BP, are measured by a LFA.

12. The method of claim 11, wherein LFA uses anti-Gal-3-BP antibodies.

13. The method of claim 12, wherein the anti-Gal-3BP antibody is SP-2 and/or any fragments thereof recognizing the same epitope.

14. The method of any one of the preceding claims, where determining Gal-3BP levels and/or Gal-3BP concentration, preferably salivary Gal-3BP levels and/or Gal-3BP concentration, in the sample comprises the steps of:
(i) incubating the sample with biotinylated and/or gold-labeled anti-Gal-3BP antibody SP-2;
(ii) determining the level/concentration of Gal-3BP by a LFA;
(iii) predicting or monitoring whether a COVID patient will progress to ARDS requiring mechanical ventilation, by evaluating whether the patient has a level of Gal-3BP > 300 ng/ml in the sample, preferably in saliva.

15. Kit for classifying a COVID-19 patient as having a risk of progressing to ARDS requiring mechanical ventilation according to any of claims 1 to 14 comprising
(i) at least one Gal-3BP targeting agent, such as an anti-Gal3BP antibody and/or antibody fragments and
(ii) a testing device for carrying out an LFA, such as a test strip.

16. The method of any of the preceding claims, wherein Gal-3BP is used as a diagnostic test companion to a therapeutic drug already commercialized or under development, in order to identify COVID-19 patients likely to respond to treatment.
